**Europäisches Patentamt**

**European Patent Office**

**Office européen des brevets**

(19)

(11) Veröffentlichungsnummer : **0 287 581 B1**

(12)

# EUROPÄISCHE PATENTSCHRIFT

(45) Veröffentlichungstag der Patentschrift :
**25.03.92 Patentblatt 92/13**

(51) Int. Cl.$^5$ : **G01N 33/53,** G01N 33/566, G01N 33/567

(21) Anmeldenummer : **87905938.4**

(22) Anmeldetag : **24.09.87**

(86) Internationale Anmeldenummer :
**PCT/CH87/00122**

(87) Internationale Veröffentlichungsnummer :
**WO 88/02861 21.04.88 Gazette 88/09**

(54) **VERFAHREN ZUR HERSTELLUNG EINER REZEPTORPRÄPARATION FÜR EINEN RADIOREZEPTOR-ASSAY UND KITGERECHTER RADIOREZEPTOR-ASSAY NACH DIESEM VERFAHREN.**

Die Akte enthält technische Angaben, die nach dem Eingang der Anmeldung eingereicht wurden und die nicht in dieser Patentschrift enthalten sind.

(30) Priorität : **13.10.86 CH 4079/86**

(43) Veröffentlichungstag der Anmeldung :
**26.10.88 Patentblatt 88/43**

(45) Bekanntmachung des Hinweises auf die Patenterteilung :
**25.03.92 Patentblatt 92/13**

(84) Benannte Vertragsstaaten :
**AT BE FR GB IT NL SE**

(56) Entgegenhaltungen :
**WO-A-86/02004
US-A- 4 162 003
US-A- 4 259 207
US-A- 4 461 829**

(73) Patentinhaber : **ANAWA LABORATORIEN AG
Unterdorfstrasse 23
CH-8602 Wangen (CH)**

(72) Erfinder : **BUERGISSER, Ernst
Unterdorfstr. 23
CH-8602 Wangen (CH)**

(74) Vertreter : **Frei, Alexandra Sarah
Frei Patentanwaltsbüro Hedwigsteig 6
Postfach 95
CH-8029 Zürich (CH)**

EP 0 287 581 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

EP 0 287 581 B1

## Beschreibung

Die Erfindung betrifft eine Einheit für einen Rezeptor-Assay im Mikromaßstab sowie ein Verfahren zur Herstellung einer solchen.

Wie beim Radioimmuno-Assay beruht das Prinzip des Radiorezeptor-Assays auf der biospezifischen Erkennung eines für den Assay gezielt eingesetzten Liganden (z.B. ein Hormon, ein Pharmakon, ein Neurotransmitter usw.) durch das entsprechende Ziel- oder Empfängermolekül. Letzteres ist beim Radioimmuno-Assay ein Antikörper, beim Radiorezeptor-Assay ein Rezeptor. Durch radioaktive Markierung des liganden erhält man ein beobachtbares Molekül, den Tracer. Dieser steht in kompetitiver Wechselwirkung mit nichtmarkierten Liganden und damit den gleichartigen aber nicht beobachtbaren Molekülen. Man erhält so ein Testsystem, welches die Messung einer unbekannten Konzentration eines solchen Liganden erlaubt.

Während der Radioimmuno-Assay zu einer viel verwendeten Routinemethode geworden ist und heute zu den in großem Maßstab verwendeten Analysemethoden gehört, wurde der Radiozeptor-Assay nur selten benutzt.

Dies liegt nicht zuletzt daran, daß die Handhabung von biologisch aktiven Rezeptoren um einiges heikler ist als die Handhabung eines Antikörpers. Voraussetzung für eine Routinemethode ist neben einer einfachen Handhabung und der damit einhergehenden Wirtschaftlichkeit vor allen Dingen die Stabilität der chemischen und biologischen Reaktionspartner und zwar sowohl für sich allein als auch insbesondere innerhalb der Analysenreaktion.

In einem Aufsatz von E. Bürgisser et al, Biochem. Biophs. Res. Commun., Band 133, Seiten 1201 bis 1209, 1985 ist die Präparation von rezeptorhaltigen Plasmamembranen beschrieben. Die in dieser Präparation enthaltenen biologisch aktiven Rezeptoren sind jedoch in Lösung instabil und müssen bis kurz vor dem analytischen Einsatz eingefroren sein, also in fester Phase vorliegen. Sind diese Bedingungen auch nur für kurze Zeit nicht erfüllt, können die Rezeptoren unbrauchbar werden. Die Plasmamembranpräparation muß daher im tiefgefrorenen Zustand auf Trockeneis versandt und bis zum Gebrauch aufbewahrt werden. Auch bei raschem Versand durch Luftfracht und sorgfältige Verpackung kann aber in der Praxis nicht gewährleistet werden, daß die Membranpräparation in einwandfreiem Zustand beim Verbraucher ankommt.

Auch die in der US-Patentschrift No. 4162003 beschriebenen Serum-Testeinheiten müssen zur Erhaltung der Aktivität der Reagentien in tiefgefrorenem Zustand gelagert und transportiert werden. Damit während der Lagerung unerwünschte Reaktionen zwischen den Reagentien vermieden werden, werden diese zwar in einem gemeinsamen Gefäss aber durch Trennelemente separat gehalten, was durch sequentielles Einfrieren oder durch Verwendung von gefriergetrockneten Reagentien möglich gemacht wird.

Durch die vorliegende Erfindung soll daher eine Einheit für einen Rezeptor-Assay im Mikromaßstab geschaffen werden, welche einfache handzuhaben ist, bei welcher kein Einfrieren von Bestandteilen der Einheit notwendig ist und bei der trotzdem die biologische Aktivität der Rezeptoren der Plasmamembranpräparation gewährleistet ist, insbesondere deren Bindungsfähigkeit.

Durch die Erfindung soll ferner ein Verfahren zur Herstellung einer derartigen Einheit angegeben werden.

Die o.g. Aufgabe ist erfindungsgemäß gelöst durch eine Einheit für einen Rezeptor-Assay im Mikromaßstab gemäß Anspruch 1.

Bei der erfindungsgemäßen Einheit liegt die Mischung der Reaktionspartner gefriergetrocknet in einem feuchtigkeitsdicht verichlossenen Röhrchen oder Näpfchen vor. Die Einheit kann ohne besondere Vorsichtsmaßnahmen auf dem Postwege versandt werden und ohne Qualitätseinbuße bei Raumtemperatur oder Kühlschranktemperatur gelagert werden. Eine ständige Kühlung durch Trockeneis oder im Gefrierschrank ist nicht notwendig.

Das Gefriertrocknen ist an sich als Verfahren zum schonenden Entwässern von Zellen und Geweben bekannt, wie das Buch "Grundriß der Gefriertrocknung" von Karlheinz Neumann, Musterschmidt-Verlag, Göttingen, 1952 zeigt. Dabei wird das gefrierzutrocknende Material vor dem Gefriertrocknen mit Glukoselösung, Lösungen von Dextrose, Maltose oder Dextran oder mit Serum oder Plasma durchtränkt. An wieder aufgetauten derartigen Präparaten konnten Lebensfunktionen beobachtet werden.

Die erfindungsgemäße Einheit für einen Rezeptor-Assay im Mikromaßstab enthält aber nicht nur eine gefriergetrocknete Präparation biologischen Materiales, sondern zugleich auch noch die anderen für das Durchführen des Rezeptor-Assays notwendigen Reaktionspartner. Diese befinden sich zusammen in dem Röhrchen oder Näpfchen, in welchem der Rezeptor-Assay durchgeführt wird. Der Assay wird dadurch in Gang gesetzt, daß mit einem einzigen Pipettierschritt die zu analysierende Lösung hinzugefügt wird.

Das Wiederaktivieren der Reaktionspartner für den Rezeptor-Assay und das Starten des Assays durch einen einzigen Pipettierschritt stellt zugleich eine erhebliche Arbeitsvereinfachung gegenüber herkömmlichen Rezeptor-Assays dar, bei denen man in der Regel drei bis vier Pipettierschritte benötigt. Die Herabsetzung der Anzahl benötigter Pipettierschritte ist zum einen im Hinblick auf die Erhöhung der Geschwindigkeit der Durch-

2

führung des Assays und im Hinblick auf den Arbeitsaufwand von Vorteil, so daß der Assay wirtschaftlicher durchgeführt werden kann. Damit erhält man auch eine erhebliche Verbesserung der Präzision der Durchführung des Assays.

Vorteilhafte Weiterbildungen der Erfindung sind in Unteransprüchen angegeben.

Mit dem im Anspruch 7 angegebenen Verfahren wird eine nicht gewünschte Vorinkubation bei der Herstellung der gefriergetrockneten Mischung der Reaktionspartner dadurch vermieden, daß die einzelnen Reaktionspartner sequentiell im Röhrchen oder Näpfchen, also im späteren Reaktionsgefäß eingefroren werden. Durch das sequentielle Tiefgefrieren wird eine Durchmischung in flüssiger Phase vermieden.

Nachstehend wird die Erfindung anhand von Ausführungsbeispielen näher beschrieben.

Für die verschiedenen unten näher angegebenen Ausführungsbeispiele wird eine Rezeptoren enthaltende Plasmamembranpräparation gemäß dem o.a. Aufsatz von E. Bürgisser et al hergestellt. Demgemäß erfolgt ein Zellaufschluß des gewünschten Gewebemateriales oder der gewünschten Zellen, z.B. Blutzellen oder Zellkulturen durch Homogenisation oder Ultraschall. Anschließend werden die zytoplasmatischen (löslichen) bestandteile und gröbere Partikel abgetrennt, z.B. durch Zentrifugieren oder Filtrieren.

Der so erhaltenen Plasmamembranpräparation wird ein zusatzstoff zugegeben, der im Hinblick auf die folgenden Eigenschaften ausgewählt ist:

1. Er soll chemisch und biochemisch inert sein, also kein Reaktionspartner des Assays sein und diesen auch nicht beeinflussen.

2. Er soll möglichst geringe oder gar keine Hygroskopizität haben.

3. Er soll in den Lösungsmitteln, die zur Auflösung des lyophilisierten Präparates verwendet werden, ebenfalls lösbar sein.

4. Fakultativ soll er ferner bewirken, daß das Lyophilisat ein eher voluminöse und kompakte Masseform annimmt, so daß es nach direkter Lyophilisierung im Reaktionsgefäß auch bei größerer mechanischer Einwirkung (Schütteln beim Postversand) fest mit der Gefäßwand verbunden bleibt.

Derartige zusatzstoffe sind: Zuckerverbindungen und deren Derivate, vorzugsweise Monosaccharide, wie Mannit, Glucose, Fructose und weitere Aldosen und Ketosen, sowie Disaccharite wie Lactose, Saccharose, sowie schwach reaktive Aminosäuren wie Glycin und/oder zusätzlich Albumine, vorzugsweise Serumalbumin vom Rind. Ferner lösliche Polysaccharide und Kollagene wie Gelatine.

Die nachstehenden Beispiele 1 bis 3 geben praktische Ausführungsbeispiele für Lösungsmittel, die für das Herstellen wässriger Lösungen von Plasmamembran geeignet sind.

Beispiel 1 (Lösungsmittel)

| | |
|---|---|
| - Tris-Puffer | 50 mM, pH 7.6 |
| - Serumalbumin vom Rind | 0,5 % |
| - D-Mannit | 2 % |
| - Stabilisatoren | Anteil gemäß System |

Die einzusetzenden Stabilisatoren sind hauptsächlich in Flüssigphase nötig, dies betrifft den Zustand der Präparation vor der Lyophilisierung und während der Inkubation in der Analyse. Bei Substanzen mit leichter Hygroskopizität besteht die Gefahr einer vorzeitigen partiellen Reaktion und/oder Degeneration im Lyophilisat. Die Stabilisatoren sind bspw. Proteaseninhibitoren, bspw. Aprotinin (Trasylol), Leupeptin etc. sowie Antioxydantien, bspw. Dithiothreitol (DDT) etc., Bakteriostatika, bspw. Natriumazid, Thimerosal und auch Komplexone, bspw. EDTA, ESTA. Ihr Einsatz hängt sehr stark vom jeweiligen Assay-System ab, das präpariert wird und sollte für jedes einzelne Testsystem evaluiert und optimiert werden. Die Kriterien dafür entsprechen der üblichen Stabilisierung von biologisch aktiven Komponenten, wie sie in den herkömmlichen Verfahren bereits benutzt werden.

Beispiel 2 (Lösungsmittel)

| | |
|---|---|
| - Phosphatpuffer | 50 mM, pH 7,4 |
| - Humanes Serumalbumin | 0,2 % |
| - Lactose | 5 % |
| - Glycerin | 0,5 |
| - Natriumazid (Stabilisator) | 0,01 % |

Beispiel 3 (Lösungsmittel)

| | |
|---|---|
| - HEPES-Puffer | 20 mH, pH 7,4 |
| - Gelatine | 1 % |
| - Glycin | 2 % |
| - Komplexon III (EDTA) | 2 mM |

Unter Verwendung eines derartigen Lösungsmittels stellt man dann Assay-Mischungen gemäß den nachstehenden Beispielen her:

Beispiel 4 (Assay-Mischung, qualitativ)

– Plasmamembranpräparation
– Tracersubstanz
– evtl. ein Standard
– evtl. Stabilisatoren und/oder Modulatoren
– evtl. Farhstoffe zur visuellen Unterscheidung von verschiedenartig präparierten Tests
– Lösungsmittel gemäß Beispiel 1 oder Beispiel 2.

Die Modulatoren beeinflussen die Bindungseigenschaften von Liganden an die Rezeptoren. Dafür werden für jeden Assay spezielle Substanzen ausgewählt. So verbessert z.B. Amilorid die Bindungsaffinität des die Natriumausscheidung in der Niere beeinflussenden, im Herzatrium hergestellten Moleküles ANP (Atrial Natriuretic Peptide, auch als ANF (Atrial Natriuretic Factor) bezeichnet) an den aus der Nebennierenrinde von Rindern gewonnenen Plasmamembranrezeptor (Lit.A.Delean, Lif.sei. 39, 1109 - 1116, 1986).

Beispiel 5 (Assay-Mischung, quantitativ)

– Plasmamembran aus der Nebennierenrinde von Rindern, 10 mg/Test biologische Ausgangsmasse
– Tracer: Jod-125 markiertes ANP, 20 000 cpm
– Standard-ANP (Verdünnungsreihe, nur in einem Teil einer Mikrotestplatte, beispielsweise in 16 von 98 Näpfchen)
– Phenantrolin (Stabilisator) 1 mM
– Farbstoff für die Markierung der Standardreihe: Evans Blue (Konzentration gemäß gewünschter Intensität).

Die einzelnen Komponenten der Assay-Mischung werden vorzugsweise in dem im Beispiel 1 angegebenen Lösungsmittel gelöst und dann sequentiell eingefroren.

Das sequentielle Einfrieren erfolgt so, daß jede im Assay reaktive Komponente für sich in dem das Testgefäß bildenden Röhrchen oder Näpfchen tiefgefroren wird, um eine Durchmischung in flüssiger Phase zu vermeiden. Zwischen zwei Schichten reaktiver Komponenten wird jeweils eine nicht-reaktive Trennschicht zwischengefroren, die aus dem im Beispiel 1 beschriebenen Lösungsmittel oder Puffersubstanz besteht. Das Einfrieren dieser Schichten erfolgt direkt in dem Testbehälter, der den alliquotierten Assay enthalten soll.

Die so erhaltene tiefgefrorene Schichtstruktur wird anschließend lyophilisiert. Das Gefriertrocknen erfolgt somit auch in den Testbehältern. Dabei wird darauf geachtet, daß sich die eingefrorenen Komponenten auch nicht kurzfristig verflüssigen können und die Lyophilisation komplett ist und keine Restfeuchtigkeit zurückbleibt.

Unmittelbar nach dem Entnehmen der Mikrotestplatten, die in der Praxis 96 Röhrchen oder Näpfchen zum Durchführen eines Radiorezeptor-Assays aufweisen, aus dem Lyophilisator werden die Testbehälter gegen Zutritt von Feuchtigkeit abgeschlossen. Vorzugsweise wird der Innenraum der Testbehälter mit einem trockenen Inertgas wie Stickstoff oder Argon gefüllt.

Mit dem oben beschriebenen Herstellungsverfahren erhält man Mikrotestplatten mit beispielsweise 96 Einheiten zur Durchführung eines Rezeptor-Assays in Mikromaßstab, die gegen das Eindringen von Feuchtigkeit z.B. mittels Klebefolien geschützt sind. Die Geometrie der Mikrotestplatten, die flache Körper sind, und die Tatsache, daß die Mikrotestplatten nicht durch Trockeneis gekühlt werden müssen, erlaubt einen problemlosen Verand als Briefpost.

In den einzelnen Röhrchen oder Näpfchen der Mikroplatte liegt die rezeptorhaltige Plasmamembranpräpartion in lyophilisierter Form vor, wobei die zugefügten Zusatzstoffe dafür sorgen, daß die gefriergetrocknete Mischung nach Rekonstitution im Assay-Puffer sofort und ohne mechanische Einwirkung wie Rühren in homogene Lösung übergeht. Das Lyophilisat zeichnet sich dadurch aus, daß seine Masse verhältnismäßig voluminös ist.

Obwohl im vorstehenden Ausführungsbeispiel auf Mikrotestplatten abgehoben wurde, bei denen die gefriergetrocknete Mischung aliquotiert in einer Vielzahl von Testbehältern enthalten ist, kann man natürlich auch entsprechende Einzel-Einheiten für einen Rezeptor-Assay herstellen, bei denen die Mischung in einem einzigen, separaten Gefäß enthalten ist.

**Patentansprüche**

1. Einheit für einen Rezeptor-Assay im Mikromaßstab, mit
a) einer gefriergetrockneten Mischung aus
aa) Plasmamembranpräparation, welche Rezeptoren enthält,
ab) einer markierten Ligandensubstanz, welche mit den Rezeptoren der Plasmamembranpräparation wechselwirken kann, und
ac) mindestens einem Zusatzstoff, der aus folgender Gruppe ausgewählt ist: Zucker, Aminosäuren, Proteine und Mischung der vorgenannten Substanzen;
b) einem Röhrchen oder Näpfchen, welches die gefriergetrocknete Mischung enthält und feuchtigkeitsdicht verschlossen ist.

2. Einheit nach Anspruch 1, bei welcher die gefriergetrocknete Mischung zusätzlich eine Vergleichssubstanz enthält.

3. Einheit nach Anspruch 1 oder 2, bei welcher die gefriergetrocknete Mischung mindestens einen Stabilisator wie Phenanthrolin enthält.

4. Einheit nach einem der Ansprüche 1 bis 3, bei welcher die gefrieroetrocknete Mischung mindestens einen Modulator enthält.

5. Einheit nach einem der Ansprüche 1 bis 4, bei welcher die gefriergetrocknete Mischung einen Farbstoff enthält.

6. Einheit nach einem der Ansprüche 1 bis 5, bei welcher die Röhrchen oder Näpfchen zu einer Mikrotestplatte gehören.

7. Verfahren zur Herstellung einer Einheit nach einem der Ansprüche 1 bis 6, bei welchem
a) eine erste Lösung hergestellt wird, die die Plasmamembranpräparation und die Zusatzstoffe enthält;
b) eine zweite Lösung hergestellt wird, die die markierte Ligandensubstanz enthält;
c) eine kleine, genau dosierte Menge einer der beiden Lösungen in das Röhrchen oder Näpfchen eingebracht und dort gefroren wird;
d) eine kleine genau dosierte Mange der anderen der beiden Lösungen über der ersten gefrorenen Schicht im Röhrchen oder Näpfchen gefroren wird;
e) die so erhaltene Schichtstruktur gefriergetrocknet wird; und
f) das Röhrchen oder Näpfchen feuchtigkeitsdicht verschlossen wird.

8. Verfahren nach Anspruch 7, bei welchem auf die gefrorene erste Schicht im Röhrchen oder Näpfchen eine nicht reaktive Trennschicht aufgefroren wird, bevor die andere der beiden Lösungen aufgefroren wird.

9. Verfahren nach Anspruch 8, bei welchem die nicht reaktive Trennschicht aus dem Lösungsmittel der ersten Lösung besteht.

10. Verfahren nach anspruch 8, bei welchem die nicht reaktive Trennschicht aus Pufferlösung besteht.

11. Verfahren nach einem der Ansprüche 7 bis 10, bei welchem eine dritte Lösung, die die Vergleichssubstanz enthält, auf die gefrorene zweite Schicht aufgefroren wird.

12. Verfahren nach Anspruch 11, bei welchem auf die zweite gefrorene Schicht eine nicht reaktive Trennschicht aufgefroren wird, bevor die dritte Lösung aufgefroren wird.

13. Verfahren nach Anspruch 11 oder 12, bei welchem die dritte Lösung zusätzlich einen Farbstoff enthält.

14. Verfahren nach einem der Ansprüche 7 bis 13, bei welchem die erste Lösung eine wässrige Mischung aus Plasmamembranpräparation und 0,1 bis 10 % w/v Mannit und/oder 0,1 bis 10 % w/v Serumalbumin von Menschen oder Rind ist.

15. Verfahren nach einem der Ansprüche 7 bis 13, bei welchem die erste Lösung eine wässrige Mischung aus Plasmamembranpräparation und 0,1 bis 10 % w/v Glycin und/oder 0,1 bis 10 % w/v Serumalbumin vom Menschen oder Rind ist.

**Claims**

1. Unit for a receptor assay in the microscale with
a) a lyophilized mixture of
aa) plasma membrane preparation containing receptors,

EP 0 287 581 B1

ab) a labelled ligand substance, which can interact with the receptors of the plasma membrane preparation and

ac) at least one additive selected from the following group: sugars, amino acids, proteins and mixtures thereof,

b) a small tube or cup containing the lyophilized mixture and which is sealed in moisture-tight manner.

2. Unit according to claim 1, wherein the lyophilized mixture additionally contains a reference substance.

3. Unit according to claims 1 or 2, wherein the lyophilized mixture contains at least one stabilizer, such as phenanthroline.

4. Unit according to one of the claims 1 to 3, wherein the lyophilized mixture contains at least one modulator.

5. Unit according to one of the claims 1 to 4, wherein the lyophilized mixture contains a dye.

6. Unit according to one of the claims 1 to 5, wherein the small tubes or cups belong to a microtest plate.

7. Process for the preparation of a unit according to one of the claims 1 to 6, wherein

a) a first solution is prepared, which contains the plasma membrane preparation and the additives,

b) a second solution is prepared, which contains the labelled ligand substance,

c) a small, precisely dosed quantity of one of the two solutions is introduced into the tube or cup and is frozen there,

d) a small, precisely dosed quantity of the other of the two solutions is frozen over the first frozen layer in the tube or cup,

e) the thus obtained layer structure is lyophilized and

f) the small tube or cup is sealed in moisture-tight manner.

8. Process according to claim 7, wherein a non-reactive separating layer is frozen onto the first frozen layer in the tube or cup, before freezing the other of the two solutions.

9. Process according to claim 8, wherein the non-reactive separating layer comprises the solvent of the first solution.

10. Process according to claim 8, wherein the non-reactive separating layer comprises the buffer solution.

11. Process according to one of the claims 7 to 10, wherein a third solution, which contains the reference substance, is frozen onto the second frozen layer.

12. Process according to claim 11, wherein a non-reactive separating layer is frozen onto the second frozen layer before the third solution is frozen on.

13. Process according to claims 11 or 12, wherein the third solution additionally contains a dye.

14. Process according to one of the claims 7 to 13, wherein the first solution is an aqueous mixture of plasma membrane preparation and 0.1 to 10% w/v of mannitol and/or 0.1 to 10% w/v of human or bovine serum albumin.

15. Process according to one of the claims 7 to 13, wherein the first solution is an aqueous mixture of plasma membrane preparation and 0.1 to 10% w/v glycine and/or 0.1 to 10% w/v human or bovine serum albumin.

**Revendications**

1. Unité pour un dosage de récepteurs à micro-échelle, comportant

(a) un mélange lyophilisé de

(aa) une préparation de membranes plasmatiques, laquelle contient des récepteurs,

(ab) une substance de ligand marquée, laquelle peut interagir avec les récepteurs de la préparation de membranes plasmatiques, et

(ac) au moins un additif, qui est choisi dans le groupe suivant : glucides, acides aminés, protéines et mélange des substances précitées ;

(b) un petit tube ou une petite coupelle, lequel ou laquelle contient le mélange lyophilisé et est fermé(e) de façon étanche à l'humidité.

2. Unité selon la revendication 1, dans laquelle le mélange lyophilisé contient en outre une substance de comparaison.

3. Unité selon l'une quelconque des revendications 1 ou 2, dans laquelle le mélange lyophilisé contient au moins un stabilisant tel que la phénanthroline.

4. Unité selon l'une quelconque des revendications 1 à 3, dans laquelle le mélange lyophilisé contient au moins un modulateur.

5. Unité selon l'une quelconque des revendications 1 à 4, dans laquelle le mélange lyophilisé contient un colorant.

6. Unité selon l'une quelconque des revendications 1 à 5, dans laquelle le petit tube ou la petite coupelle appartient à une plaque de microtitration.

7. Procédé de fabrication d'une unité telle que définie à l'une quelconque des revendications 1 à 6, dans

6

lequel

(a) une première solution est préparée, laquelle contient la préparation de membranes plasmatiques et les additifs;

(b) une deuxième solution est préparée, laquelle contient la substance de ligand marquée ;

(c) une petite quantité, dosée de façon précise, de l'une des deux solutions est placée dans le petit tube ou la petite coupelle et elle y est congelée ;

(d) une petite quantité, dosée de façon précise, de l'autre des deux solutions est congelée sur la première couche congelée dans le tube ou la coupelle;

(e) la structure en couches ainsi obtenue est lyophilisée ; et

(f) le petit tube ou la petite coupelle est obturé(e) de façon étanche à l'humidité.

8. Procédé selon la revendication 7, dans lequel, sur la première couche congelée dans le petit tube ou la petite coupelle, est congelée une couche de séparation non-réactive, avant que l'autre des deux solutions ne soit congelée.

9. Procédé selon la revendication 8, dans lequel la couche de séparation non-réactive se compose du solvant de la première solution.

10. Procédé selon la revendication 8, dans lequel la couche de séparation non-réactive se compose d'une solution tampon.

11. Procédé selon l'une quelconque des revendications 7 à 10, dans lequel une troisième solution, qui contient la substance de comparaison, est congelée sur la deuxième couche congelée.

12. Procédé selon la revendication 11, dans lequel, sur la deuxième couche congelée, est congelée une couche de séparation non-réactive, avant que la troisième solution ne soit congelée.

13. Procédé selon l'une quelconque des revendications 11 ou 12, dans lequel la troisième solution contient en outre un colorant.

14. Procédé selon l'une quelconque des revendications 7 à 13, dans lequel la première solution est un mélange aqueux d'une préparation de membranes plasmatiques et de 0,1 à 10 % p/v de mannitol et/ou de 0,1 à 10 % p/v de sérum albumine humaine ou bovine.

15. Procédé selon l'une quelconque des revendicacation 7 à 13, dans lequel la première solution est un mélange aqueux d'une préparation de membranes plasmatiques et de 0,1 à 10 % p/v de glycine et/ou de 0,1 à 10 % p/v de sérum albumine humaine ou bovine.